# NEW EUROPEAN PATENT SPECIFICATION

(11) **EP 1 417 946 B2**
(45) Date of publication and mention of the opposition decision: **15.12.2010**
(45) Mention of the grant of the patent: 19.04.2006
(21) Application number: 03026003.8
(22) Date of filing: 11.11.2003
(51) Int. Cl.: A61F 13/15, D04H 1/50

(54) **Absorbent article and process for producing an absorbent member for an absorbent article**
Absorbierender Artikel und Verfahren zur Herstellung einer absorbierenden Einheit für einen absorbierenden Artikel
Article absorbant et procédé de fabrication d' un corps absorbant pour article absorbant

(30) Priority: 11.11.2002 JP 2002327355
(43) Date of publication of application: 12.05.2004
(73) Proprietor: KAO CORPORATION, Tokyo (JP)
(72) Inventor: Nakayama, Takao, Kao Corporation, Haga-gun Tochigi (JP); Toyoshima, Yasuo, Kao Corporation, Haga-gun Tochigi (JP); Nagahara, Shinsuke, Kao Corporation, Haga-gun Tochigi (JP)
(74) Representative: Vossius & Partner

(56) References cited:
- EP-A- 0 729 735
- EP-A- 1 247 509
- WO-A-01/47456
- WO-A-93/15249
- JP-A- 05 140 848
- JP-A- 2002 285 464
- US-A- 5 681 300
- US-A- 5 683 374
- US-A- 5 891 119
- PATENT ABSTRACTS OF JAPAN vol. 017, no. 523 (C-1113), 21 September 1993 (1993-09-21) & JP 05 140848 A (KAO CORP), 8 June 1993 (1993-06-08)
- PATENT ABSTRACTS OF JAPAN vol. 018, no. 619 (C-1278), 25 November 1994 (1994-11-25) & JP 06 237956 A (UNI CHARM CORP), 30 August 1994 (1994-08-30)
- PATENT ABSTRACTS OF JAPAN vol. 014, no. 261 (C-0725), 6 June 1990 (1990-06-06) & JP 02 074254 A (UNI CHARM CORP), 14 March 1990 (1990-03-14)

## Description

The present invention relates to an absorbent article such as a sanitary napkin a panty liner or an incontinence pad. More particularly, it relates to an absorbent article in which an absorbent member is prevented from bunching up or twisting, and has increased absorptivity, a better fit and improved comfort. The present invention further relates to a process of producing an absorbent member for an absorbent article.

It is known that an absorbent member of a body fluid absorbent article can be made of a mixture of helically crimped thermoplastic fiber and ground pulp in order for the absorbent member to have elasticity and a soft feel (see JP-A-6-237956).

It is also known to make an absorbent mat of a mixture of thermally fusible crimped fiber, fluffy pulp, and absorbent, polymer particles in order to obtain an absorbent member that has reduced bulkiness before absorbing body fluid but, upon absorption, is restored to gain in bulkiness, elasticity, and absorption capacity (see JP-B-6-38814).

Both the above-described absorbent members are fabricated using previously crimped fibers as a raw material. Therefore, the crimped fiber contained in the absorbent member is incapable of keeping the absorbent member sufficiently bulky (incapable of preventing the absorbent member from losing its thickness and cushioning properties) when the hydrophilic fiber, e.g., pulp, absorbs liquid. Consequently, the absorbent member after liquid absorption bunches easily and tends to largely lose its bulkiness and ability to fit properly on the wearer, compared with the member before liquid absorption. Wet-back occurs easily for the same reasons.

In JP-A-05/140848 a certain water-absorbing structure and its production is described. US-A-5,891,119 relates to a certain absorptive article comprising a liquid-permeable front sheet, a liquid-impermeable back sheet and an absorbent placed between the two sheets, this absorbent being composed of at least two layers.

The present invention relates to absorbent articles as described and claimed in claims 1 and 2.

The present invention also provides processes of producing absorbent members for absorbent articles as described and claimed in claims 6 and 7.

While the specification concludes with claims particularly pointing out and distinctly claiming the subject matter which is regarded as forming the present invention, it is believed that the invention will be better understood from the following description which is taken in conjunction with the accompanying drawings and in which:
Fig. 1 is a perspective view of a sanitary napkin as an embodiment of the absorbent article according to the present invention, with a part cut away;
Fig. 2 is a schematic cross-sectional view of the absorbent member used in the napkin of Fig. 1; and
Fig. 3 is a schematic cross-sectional view (corresponding to Fig. 2) of an absorbent member according to a second embodiment of the present invention.

The present invention relates to an absorbent article in which an absorbent member is prevented from bunching or twisting, and has increased absorptivity, a better fit and improved comfort.

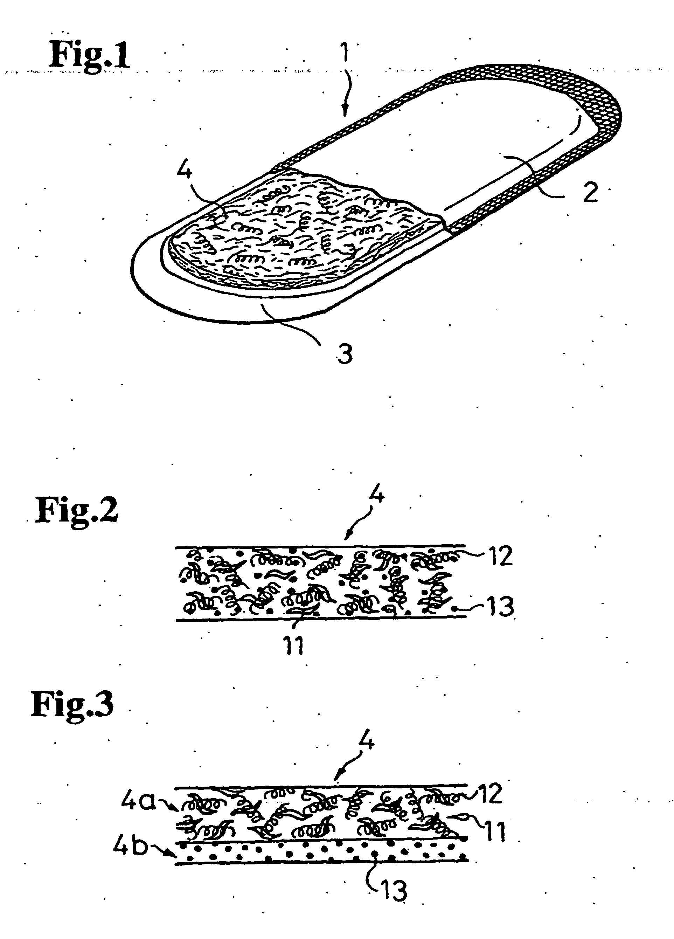

The present invention will be described based on its preferred embodiments while referring to the accompanying drawings. Fig. 1 is a perspective view of a sanitary napkin as a preferred embodiment of the absorbent article according to the present invention, with a part cut away. Fig. 2 is a schematic cross-section of the absorbent member used in the napkin of Fig. 1. The sanitary napkin 1 according to this embodiment has an oblong shape and comprises a liquid permeable topsheet 2, a liquid impermeable backsheet 3, and an absorbent member 4 as a liquid retentive absorbing layer interposed between the sheets 2 and 3. The topsheet 2 and the backsheet 3 extend over the lateral sides and the longitudinal ends of the absorbent member 4 and are joined to each other at the extensions by joining means, such as a heat seal or a hot-melt adhesive.

As illustrated in Fig. 2, the absorbent member 4 is composed of hydrophilic fibers 11, helically crimped synthetic resin fibers 12 (hereinafter simply referred to as "helically crimped fiber(s)"), and superabsorbent polymer particles 13. The presence of the helically crimped fibers 12 makes the absorbent member 4 bulky. When compressed, the helically crimped fibers 12 have a nature of exhibiting resistance and restoring their original state, whereby the absorbent member 4 has satisfactory cushioning properties and elasticity in the thickness direction. As a result, the napkin 1 as a whole keeps a satisfactory feel including a cushioning feel and gives a wearer comfort while being worn. Furthermore, owing to the recovering nature of the helically crimped fibers 12 from compression, the napkin 1 is prevented from bunching up or twisting even with an outer force applied thereto by the wearer's movement while being worn. In particular, bunching of the napkin 1 in the lateral direction is prevented,

The helically crimped fiber 12 embraces at least one, preferably two or more hydrophilic fibers 11 in its helix. The crimped fiber 12 also preferably embraces at least one, preferably two or more superabsorbent polymer particles 13 per helix. When seen microscopically, the absorbent member 4 is made up of numerous composite fiber structures each composed of a helically crimped fiber 12, a hydrophilic fiber 11 and/or a superabsorbent polymer particle 13. The composite fiber structures are almost uniformly distributed in the absorbent member 4. When the hydrophilic fiber 11 absorbs liquid, the hydrophilic fibers 11 are prevented from getting close to one another by the embrace of the helically crimped fiber 12 so that the absorbent member 4 is effectively prevented from losing its bulkiness. The hydrophilic fiber 11 embraced in the helix of the helically crimped fiber 12 provides space within the helically crimped fiber 12. Therefore, when the superabsorbent polymer particle 13 swells on absorption, gel blocking hardly occurs, and absorptivity is improved. Since the superabsorbent polymer particles 13 are preferably embraced in the helices of the helically crimped fibers 12, the superabsorbent polymer particles 13 hardly fall out even when the suberabsorbent polymer particles 13 are used at an increased amount. It should be noted that all the hydrophilic fibers 11 are not always embraced in the helices of the helically crimped fibers 12. Similarly, all the superabsorbent particles 13 are not always embraced in the helices of the helically crimped fibers 12.

The helically crimped fiber 12 is preferably a self-crimping fiber having been crimped in a helical configuration on being heated. The self-crimping fibers are shrunken and crimped while embracing the hydrophilic fibers 11 and/or the superabsorbent polymer particles 13 in their helices to form composite fiber structures. Due to the crimping and shrinking of the helically crimped fiber 12, the hydrophilic fibers 11 and/or the superabsorbent polymer particles 13 are embraced therein. The helically crimped fibers 12 can be dispersed uniformly throughout the absorbent member in a manner commonly employed in the production of general absorbent members (for example an air-laying method) because the fibers have a latent crimp before being crimped, i.e., the self-crimping fibers can be handled in the same manner as for ordinary fibers. Thereafter, the self-crimping fibers are preferably thermally crimped to form a great number of composite fiber structures containing the helically crimped fiber 12, which are distributed evenly throughout the absorbent member. Thus, the resulting absorbent member 4 exhibits stabilized absorbing performance and sufficient elasticity owing to the helically crimped fiber 12. The stabilized absorbing performance and sufficient elasticity greatly contribute to preventing the absorbent member 4 from bunching and to improving comfort such as having a soft feel. They also greatly contribute to improving the absorption characteristics including the amount and the rate of absorption and to reduce wet-back. They also contribute to improvement of fit, i.e., conformability to a wearer's movement

In order to effectively obtain the above-mentioned effects of the helically crimped fiber 12, it is preferred for the absorbent member 4 to contain from 40 to 90%, preferably from 50 to 90%, by weight of the crimped fiber 12. For the same purpose, the helically crimped fiber 12 has 5 to 15 crimps per inch. The number of crimps is measured in accordance with JIS L1015. For the same purpose, it is preferred for the helically crimped fiber 12 to have a fineness of from 1 to 15 dtex, more preferably from 2 to 10 dtex. The helically crimped fiber 12 is not particularly limited in its contour and may have, for example, a circular section, an elliptic section or a dumbbell-shaped section.

The hydrophilic fiber 11 is preferably present in the absorbent member 4 in an amount of from 10 to 60%, more preferably from 10 to 50%, by weight so that it may retain liquid among fibers (part of it takes in liquid inside the fibers) to lower the freedom of the liquid. The superabsorbent polymer particles 13 are present in the absorbent member 4 in an amount of from 3 to 20%, more preferably from 5 to 15%, by weight so that an appropriate amount of the superabsorbent polymer may be caught up by the helically crimped fiber 12 to bring about improved absorbing performance. With part of, for example 20% or more by weight of, the superabsorbent polymer particles 13 being caught and embraced by the helically crimped fiber 12, a gel blocking phenomenon is effectively averted, and the absorptivity is improved.

The absorbent member 4 contains thermally fusible fibers. Thermally fusible fibers incorporated into the absorbent member 4 are fused to each other to provide a network structure in the absorbent member 4. The network structure of fusible fibers makes the absorbent member 4 stronger and prevents the absorbent member 4 from bunching. Similarly to the hydrophilic fiber, the thermally fusible fiber is embraced in the helically crimped fiber 12, whereby the aforementioned numerous composite fiber structures are indirectly linked via the thermally fusible fiber to stabilize the network structure of the thermally fusible fiber. As a result, the elastic extensibility and contractibility of the absorbent member are further improved. A preferred content of the thermally fusible fiber in the absorbent member 4 is from 10 to 30%, more preferably from 15 to 25%; by weight for improving the strength of the absorbent member 4 and preventing the napkin 1 from bunching. Useful thermally fusible fibers include solid or hollow sheath/core type fibers.

Where, in particular, the absorbent member 4 contains the helically crimped fiber 12 in a relatively high proportion, for example from 60 to 90% by weight of the helically crimped fiber 12 with from 10 to 40% by weight of the hydrophilic fiber 11, there will be increased probability for a helically crimped fiber 12 to be embraced in another helically crimped fiber 12. As a result, the composite fiber structures have increased elasticity, and the absorbent member 4 provides a further improved fit. Because the composite fiber structures are linked via helically crimped fibers 12, the network structure of the thermally fusible fiber is further stabilized. As a result, the absorbent member 4 shows further improved elasticity in extension and contraction, which provides the sanitary napkin 1 with improved flexibility and a better fit. In addition, the absorbent member 4 allows liquid to pass more quickly (the rate of absorption increases) and suppresses wet-back more effectively.

As previously stated, the presence of the helically crimped fiber 12 makes the absorbent member 4 bulky. Because of this bulkiness, the absorbent member 4 has a reduced density. Specifically, the absorbent member 4 has a preferred density of from 0.03 to 0.2 g/cm³, more preferably from 0.03 to 0.15 g/cm³. While subject to variation according to the intended use of the sanitary napkin 1 (for night application, light days application, etc.), the thickness of the absorbent member 4 is preferably about from 1 to 3 mm. Accordingly, the basis weight of the absorbent member 4 preferably would be as small as about 30 to 150 g/m², more preferably about 40 to 70 g/m².

The absorbent member 4 preferably has a bending rigidity of 0.02 to 0.3 gf·cm/cm², more preferably 0.02 to 0.15 gf·cm/cm², for securing improved flexibility and reducing discomfort of the napkin while worn. The absorbent member 4 with such a bending rigidity is easily obtained by using helically crimped fiber 12 as the constituent material of the absorbent member 4 and entangling the hydrophilic fiber 11 in the helix of the helically crimped fiber 12.

The bending rigidity is measured as follows. The measurement was made according to the system described in Sueo Kawabata, Fuaihyouka no Kyojunka to Kaiseki (2nd Ed.), Hand Measurement and Standardization Research Group in The Textile Machinery Society of Japan (7/10/1980), pp. 27-28. In detail, an absorbent member is cut to a length of at least 20 mm in the with direction and at least 20 mm in longitudinal direction to prepare a specimen. The specimen is clamped in the jaws of a pure bending tester (KES-FB2, manufactured by Kato Tech Co., Ltd.) set at a distance of 10 mm with the longitudinal direction of the absorbent member being a bending direction. To minimize the influence of gravity, the specimen is clamped vertically. The specimen is bent at a constant rate of curvature change within a curvature range K of from -2.5 to +2.5 cm⁻¹. The rate of deformation is 0.50 cm⁻¹/sec. The relationship between bending moment M per unit area and curvature K (M-K curve) is obtained, from which a bending rigidity B per unit length (gf·cm²/cm), the slope of the M-K curve, is calculated as follows. The slope Bf between K=0.5 cm⁻¹ and 1.5 cm⁻¹ and the slope Bb between K=-0.5 cm⁻¹ and -1.5 cm⁻¹ are measured from the characteristics in the curves with the absolute K value increasing. An average of Bf and Bb, (Bf+Bf)/2, is taken as a bending rigidity. The smaller the bending rigidity, the softer the absorbent member.

Materials making the absorbent member 4 will be described in the following. The helically crimped fiber 12 is preferably a self-crimping fiber having a latent crimp as previously stated. The self-crimping fiber includes eccentric sheath/core conjugate fibers and side-by-side conjugate fibers. The self-crimping fiber can have a fiber length of choice according to the method of fabricating the absorbent member 4. Where the absorbent member 4 is fabricated by an air-laying method, for example, the fiber length of the self-crimping fiber is preferably about 32 to 75 mm. After self-crimping, the fiber preferably has an apparent fiber length of about 40 to 70% of that before crimping. Resins which constitute the self-crimping fiber include polyethylene, polypropylene, polyesters (e.g., polyethylene terephthalate), and ethylene-vinyl alcohol copolymers. The self-crimping fiber preferably has its surface hydrophilized with a hydrophilizing agent such as a surface active agent.

The hydrophilic fiber 11 is preferably one having no fusibility, such as pulp, rayon or cotton. These fibers can have a fiber length of choice according to the method of making the absorbent member 4. Where the absorbent member 4 is fabricated by an air-laying method, for example, the hydrophilic fiber preferably has a fiber length of about 32 to 75 mm. The superabsorbent polymer includes poly(sodium acrylate), acrylic add-vinyl alcohol copolymers, crosslinked poly(sodium acrylate), starch-acrylic acid graft polymers, and isobutylene-maleic anhydride copolymers. The superabsorbent polymer particles 13 preferably have an average particle size of about 100 to 300 µm.

In the following a preferred process of making the absorbent member 4 used in the napkin 1 according to the present embodiment is described. A self-crimping fiber, hydrophilic fiber, a thermally fusible fiber and, optionally, superabsorbent polymer particles are used as raw materials. These materials are carried in an air stream and accumulated by an air-laying system to form a web. Heat is applied to the web to cause the self-crimping fiber to develop helical crimps. Heat application is carried out by hot air blowing, infrared irradiation or like means. Hot air blowing is preferred.

In the web, the materials are in a mixed state. The self-crimping fiber develops crimps in this state. It follows that the helically crimped fiber embraces part of the hydrophilic fiber, part of the thermally fusible fiber and part of the superabsorbent polymer particles, if used, in its helix. Where the self-crimping fiber is used in a higher proportion, the helix of a single crimped fiber will embrace another crimped fiber at a higher probability thereby to provide a network structure of the helical fibers. Heat-application results in formation of fused intersections of the fusible fibers thereby providing a network structure of the fusible fiber.

A second embodiment of the present invention will be described by referring to Fig. 3. The second embodiment is described with reference only to the differenced from the first one. The explanation given to the first embodiment applies to the second one with respect to those particulars that are not described here. The members common to Figs. 2 and 3 are given the same reference numerals.

In this embodiment, the absorbent member 4 has a double layer structure. In detail, the absorbent member 4 has an upper layer 4a located nearer to a wearer and a lower layer 4b located under the upper layer 4a. The upper layer 4a contains hydrophilic fiber 11 and helically crimped fiber 12. The helically crimped fiber 12 embraces one or more hydrophilic fibers per helix to provide a large number of composite fiber structures. The composite fiber structures are distributed substantially uniformly throughout the upper layer 4a. The network of the composite fiber structures can be formed easily by increasing the proportion of the helically crimped fiber 12 in the upper layer 4a.

The ratio of the helically crimped fiber 12 and the hydrophilic fiber 11 in the upper layer 4a is the same as in the first embodiment. The upper layer 4a contains thermally fusible fiber forming a network structure by heat fusion. The ratio of the thermally fusible fiber can be the same as in the first embodiment.

The lower layer 4b comprises superabsorbent polymer particles 13. The ratio of the superabsorbent polymer particles 13 is the same as in the first embodiment. The lower layer 4b may contain hydrophilic fiber. Where the proportion of the hydrophilic fiber is higher in the lower layer 4b than in the upper layer 4a, liquid is more easily led into the lower layer 4b. Thermally fusible fiber may be incorporated into the lower layer 4b to form a network structure of the thermally fusible fiber or to be fusion-bonded to the thermally fusible fiber present in the upper layer 4a, thereby improving integrity of the absorbent member 4 and preventing the absorbent member 4 from bunching.

In the absorbent member 4 of a sanitary napkin according to this embodiment, the upper layer 4a contributes to increasing the rate of liquid migration, and the lower layer 4b contributes to liquid absorptivity and retention. That is, the functions required of an absorbent member are separately performed, which results in an advantage that the freedom of designing an absorbent member increases. In view of the extensibility of the helically crimped fiber 12, there is also obtained another advantage that the absorbent member 4 provides an improved fit.

The absorbent member 4 of a sanitary napkin according to the second embodiment is preferably prepared by the following method. Self-crimping fiber, hydrophilic fiber, and thermally fusible fiber are used as raw materials. These fibers are accumulated by an air-laying system to form a web. Heat is applied to the web to cause the self-crimping fiber to develop helical crimps. Since the self-crimping fiber and the hydrophilic fiber are in a uniformly mixed state in the web, the self-crimping fibers develop crimps while embracing the hydrophilic fiber in their helices. Where the self-crimping fiber is used in a higher proportion, the helix of a single crimped fiber will embrace another crimped fiber at a higher probability. Heat application results in formation of fused intersections of the thermally fusible fibers. The upper layer 4a is thus formed through these steps. Subsequently, the resulting web (upper layer 4a) is superposed on a layer of superabsorbent polymer particles (i.e., a lower layer 4b) previously prepared in a separate accumulation step to obtain the absorbent member 4.

The present invention is not limited to the above-described embodiments. For instance, the absorbent member 4 used in the above-described embodiments, which has a single or a double layer structure, can be replaced with an absorbent member having three or more layers. While the foregoing embodiments employ thermally fusible fiber in combination with helically self-crimping fiber, the helically self-crimping fiber can also serve as thermally fusible fiber.

Applications of the absorbent article of the present invention are not limited to sanitary napkins and additionally include other absorbent articles such as panty liners, incontinence pads, and disposable diapers.

As described in detail, the absorbent article according to the present invention is free from the absorbent member's bunching problem and provides increased absorptivity, improved comfort, and a better fit.

## Claims

1. An absorbent article comprising an absorbent member, wherein the absorbent member comprises:
(i) hydrophilic fibers,
(ii) helically crimped synthetic resin fibers having 5 to 15 crimps per inch (5 to 15 crimps per 2.54 cm),
(iii) thermally fusible fibers, and
(iv) 3 to 20 % by weight superabsorbent polymer particles,
wherein the hydrophilic fibers, the thermally fusible fibers and the superabsorbent polymer particles are embraced in the helices of the helically crimped fibers, thereby forming composite fiber structures, wherein a plurality of the composite fiber structures are distributed throughout the absorbent member substantially uniformly, and wherein the thermally fusible fibers are fused to each other to provide a network structure.

2. An absorbent article comprising an absorbent member, wherein the absorbent member comprises:
(i) hydrophilic fibers,
(ii) helically crimped synthetic resin fibers, having 5 to 15 crimps per inch (5 to 15 crimps per 2.54 cm)
(iii) thermally fusible fibers, and
(iv) 3 to 20% by weight superabsorbent polymer particles,
wherein the hydrophilic fibers and the thermally fusible fibers are embraced in the helices of the helically crimped fibers, thereby forming composite fiber structures, wherein a plurality of the composite fiber structures are distributed throughout the absorbent member substantially uniformly, and wherein the thermally fusible fibers are fused to each other to provide a network structure,
wherein further the absorbent member comprises an upper layer located nearer to a wearer and a lower layer located under the upper layer, wherein the upper layer contains the composite fiber structures, which are distributed substantially uniformly throughout the upper layer, and wherein the lower layer contains the superabsorbent polymer particles.

3. The absorbent article according to claim 1 or 2, wherein the helically crimped fiber is a self-crimping eccentric sheath/core conjugate fiber having a latent crimp or a side-by-side conjugate fiber having a latent crimp which has been crimped in a helical configuration by heat application while embracing the hydrophilic fiber in the helix thereof.

4. The absorbent article according to any one of claims 1 to 3, wherein the absorbent member comprises from 10 to 60% by weight of the hydrophilic fibers and from 40 to 90% by weight of the helically crimped fibers, whereby the helically crimped fiber embraces another helically crimped fiber in the helix thereof.

5. The absorbent article according to any one of claims 1 to 4, wherein the absorbent member comprises from 10 to 30% by weight of thermally fusible fibers.

6. A process of producing an absorbent member for an absorbent article according to claim 1, which comprises the steps of:
carrying the hydrophilic fibers, self-crimping fibers having a latent crimp, the thermally fusible fibers and the superabsorbent polymer particles in an air stream and accumulating by an air-laying system to form a web and
applying heat to the web to cause the self-crimping fibers to develop helical crimps while embracing the hydrophilic fibers, the thermally fusible fibers and the superabsorbent polymer particles in their helices to form composite fiber structures and to cause the thermally fusible fibers to be heat fused to each other thereby to form a network structure.

7. A process of producing an absorbent member according to claim 2, which comprises the steps of:
carrying the hydrophilic fibers, self-crimping fibers having a latent crimp and the thermally fusible fibers in an air stream and accumulating by an air-laying system to form a web, and
applying heat to the web to cause the self-crimping fibers to develop helical crimps while embracing the hydrophilic fibers and the thermally fusible fibers in their helices to form composite fiber structures and to cause the thermally fusible fibers to be heat fused to each other thereby to form a network structure.

## Patentansprüche

1. Ein absorbierender Gegenstand, umfassend ein absorbierendes Element, wobei das absorbierende Element umfasst:
(i) hydrophile Fasern,
(ii) helixförmig gekräuselte synthetische Harzfasern mit 5 bis 15 Kräuselungen pro Zoll (5 bis 15 Kräuselungen pro 2,54 cm),
(iii) thermisch schmelzbare Fasern und
(iv) 3 bis 20 Gew.-% superabsorbierende Polymerteilchen,
wobei die hydrophilen Fasern, die thermisch schmelzbaren Fasern und die superabsorbierenden Polymerteilchen von den Helices der helixförmig gekräuselten Fasern umspannt sind, und somit zusammengesetzte Faserstrukturen bilden, wobei eine Mehrzahl der zusammengesetzten Faserstrukturen im Wesentlichen einheitlich überall in dem absorbierenden Element verteilt sind, und wobei die thermisch schmelzbaren Fasern miteinander verschmolzen sind, um eine Netzwerkstruktur bereitzustellen.

2. Ein absorbierender Gegenstand umfassend ein absorbierendes Element, wobei das absorbierende Element umfasst:
(i) hydrophile Fasern,
(ii) helixförmig gekräuselte synthetische Harzfasern mit 5 bis 15 Kräuselungen pro Zoll (5 bis 15 Kräuselungen pro 2,54 cm),
(iii) thermisch schmelzbare Fasern und
(iv) 3 bis 20 Gew.-% superabsorbierende Polymerteilchen,
wobei die hydrophilen Fasern und die thermisch schmelzbaren Fasern von den Helices der helixförmig gekräuselten Fasern umspannt sind, und somit zusammengesetzte Faserstrukturen bilden, wobei eine Mehrzahl der zusammengesetzten Faserstrukturen im Wesentlichen einheitlich überall in dem absorbierenden Element verteilt sind, und wobei die thermisch schmelzbaren Fasern miteinander verschmolzen sind, um eine Netzwerkstruktur bereitzustellen,
wobei das absorbierende Element weiter eine obere Schicht, die sich näher am Träger befindet, und eine untere Schicht, die sich unter der oberen Schicht befindet, umfasst, wobei die obere Schicht die zusammengesetzten Faserstrukturen enthält, die im Wesentlichen einheitlich überall in der oberen Schicht verteilt sind, und wobei die untere Schicht die superabsorbierenden Polymerteilchen enthält.

3. Der absorbierende Gegenstand nach Anspruch 1 oder 2, wobei die helixförmig gekräuselte Faser eine selbstkräuselnde exzentrische Mantel/Kern-Konjugatfaser mit einer latenten Kräuselung ist oder eine Seite-an-Seite Konjugatfaser mit einer latenten Kräuselung ist, die durch Wärmeanwendung zu einer helixförmigen Anordnung gekräuselt wurde, während sie die hydrophile Faser in der Helix davon umspannte.

4. Der absorbierende Gegenstand nach einem der Ansprüche 1 bis 3, wobei das absorbierende Element 10 bis 60 Gew.-% der hydrophilen Fasern und 40 bis 90 Gew.-% der helixförmig gekräuselten Fasern umfasst, wobei die helixförmig gekräuselte Faser eine andere helixförmig gekräuselte Faser in der Helix davon umspannt.

5. Der absorbierende Gegenstand nach einem der Ansprüche 1 bis 4, wobei das absorbierende Element 10 bis 30 Gew.-% an thermisch schmelzbaren Fasern umfasst.

6. Ein Verfahren zur Herstellung eines absorbierenden Elements für einen absorbierenden Gegenstand nach Anspruch 1, wobei das Verfahren die Schritte umfasst:
Befördern der hydrophilen Fasern, der selbstkräuselnden Fasern mit einer latenten Kräuselung, der thermisch schmelzbaren Fasern und der superabsorbierenden Polymerteilchen in einem Luftstrom und Sammeln durch ein Luftlegungssystem, um eine Bahn zu bilden, und
Aufbringen von Wärme auf die Bahn, damit die selbstkräuselnden Fasern helixförmige Kräuselungen entwickeln, während die hydrophilen Fasern, die thermisch schmelzbaren Fasern und die superabsorbierenden Polymerteilchen von ihren Helices umspannt sind,
um zusammengesetzte Faserstrukturen zu bilden und damit die thermisch schmelzbaren Fasern miteinander thermisch verschmelzen, um somit eine Netzwerkstruktur zu bilden.

7. Ein Verfahren zur Herstellung eines absorbierenden Elements nach Anspruch 2, wobei das Verfahren die Schritte umfasst:
Befördern der hydrophilen Fasern, der selbstkräuselnden Fasern mit einer latenten Kräuselung und der thermisch schmelzbaren Fasern in einem Luftstrom und Sammeln durch ein Luftlegungssystem, um eine Bahn zu bilden, und
Aufbringen von Wärme auf die Bahn, damit die selbstkräuselnden Fasern helixförmige Kräuselungen entwickeln, während die hydrophilen Fasern und die thermisch schmelzbaren Fasern von ihren Helices umspannt sind, um zusammengesetzte Faserstrukturen zu bilden und damit die thermisch schmelzbaren Fasern miteinander thermisch verschmelzen, um somit eine Netzwerkstruktur zu bilden.

## Revendications

1. Article absorbant comprenant un corps absorbant, dans lequel le corps absorbant comprend:
(i) des fibres hydrophiles;
(ii) des fibres en résine synthétique frisées de manière hélicoïdale ayant de 5 à 15 frisures par pouce (de 5 à 15 frisures par 2,54 cm);
(iii) des fibres thermiquement fusibles; et
(iv) de 3 à 20 % en poids de particules polymères superabsorbantes,
dans lequel les fibres hydrophiles, les fibres thermiquement fusibles et les particules polymères superabsorbantes sont englobées dans les helices des fibres frisées de manière hélicoïdale, formant ainsi des structures de fibres composites, dans lequel une pluralité des structures de fibres composites sont distribuées dans tout le corps absorbant de manière sensiblement uniforme, et dans lequel les fibres thermiquement fusibles sont fondues les unes avec les autres pour fournir une structure de réseau.

2. Article absorbant comprenant un corps absorbant, dans lequel le corps absorbant comprend:
(i) des fibres hydrophiles;
(ii) des fibres en résine synthétique frisées de manière hélicoïdale ayant de 5 à 15 frisures par pouce (de 5 à 15 frisures par 2,54 cm);
(iii) des fibres thermiquement fusibles; et
(iv) de 3 à 20 % en poids de particules polymères superabsorbantes,
dans lequel les fibres hydrophiles et les fibres thermiquement fusibles sont englobées dans les helices des fibres frisées de manière hélicoïdale, formant ainsi des structures de fibres composites, dans lequel une pluralité des structures de fibres composites sont distribuées dans tout le corps absorbant de manière sensiblement uniforme, et dans lequel les fibres thermiquement fusibles sont fondues les unes avec les autres pour fournir une structure de réseau,
dans lequel le corps absorbant comprend en outre une couche supérieure située plus près d'un porteur et une couche inférieure située sous la couche supérieure, dans lequel la couche supérieure contient les structures de fibres composées qui sont distribuées de manière sensiblement uniforme dans toute la couche supérieure, et dans lequel la couche inférieure contient les particules polymères superabsorbantes.

3. Article absorbant selon la revendication 1 ou 2, dans lequel la fibre frisée de manière hélicoïdale est une fibre conjugée gaine/âme enrobée excentrique autofrisable ayant une frisure latente ou une fibre conjuguée côte à côte ayant une frisure latente, qui a été frisée en une configuration hélicoïdale par application thermique tout en englobant la fibre hydrophile dans son hélice.

4. Article absorbant selon l'une quelconque des revendications 1 à 3, dans lequel le corps absorbant comprend de 10 à 60 % en poids de fibres hydrophiles et de 40 à 90 % en poids de fibres frisées de manière hélicoïdale, dans lequel la fibre frisée de manière hélicoïdale englobe une autre fibre frisée de manière hélicoïdale dans son hélice.

5. Article absorbant selon l'une quelconque des revendications 1 à 4, dans lequel le corps absorbant comprend de 10 à 30 % en poids des fibres thermiquement fusibles.

6. Procédé de fabrication d'un corps absorbant pour un article absorbant selon la revendication 1, comprenant les étapes consistant à:
porter les fibres hydrophiles, les fibres autofrisables ayant une frisure latente, les fibres thermiqument fusibles et les particules polymères superabsorbantes dans un courant d'air et les accumuler par un système de formation par flux d'air pour former une toile; et
appliquer de la chaleur à la toile pour faire developer des frisures hélicoïdales par les fibres autofrisables tout en englobant les fibres hydrophiles, les fibres thermiquement fusibles et les particules polymères superabsorbantes dans leurs helices pour former des structures de fibres composites et pour causer la fusion thermique des fibres thermiquement fusibles entre elles pour former ainsi une structure de réseau.

7. Procédé de fabrication d'un corps absorbant selon la revendication 2, comprenant les étapes consistant à:
porter les fibres hydrophiles, les fibres autofrisables ayant une frisure latente et les fibres thermiquement fusibles dans un courant d'air et les accumuler par un système de formation par flux d'air pour former une toile; et
appliquer de la chaleur à la toile pour faire developer des frisures hélicoïdales par les fibres autofrisables tout en englobant les fibres hydrophiles et les fibres thermiquement fusibles dans leurs helices pour former des structures de fibres composites et pour causer la fusion thermique des fibres thermiquement fusibles entre elles pour former ainsi une structure de réseau.
